# EUROPEAN PATENT SPECIFICATION

(11) **EP 2 781 225 B1**
(45) Date of publication and mention of the grant of the patent: **09.10.2019**
(21) Application number: 12849808.6
(22) Date of filing: 16.11.2012
(51) Int. Cl.: A61K 47/69, A61K 47/54, A61K 45/08, A61P 1/04, C12N 5/071, C12N 15/09, G01N 33/15, G01N 33/50, A61K 9/127, A61K 31/713

(54) **INTESTINAL FIBROSIS TREATMENT AGENT**
BEHANDLUNGSMITTEL FÜR INTESTINALE FIBROSE
AGENT DE TRAITEMENT DE LA FIBROSE INTESTINALE

(30) Priority: 18.11.2011 JP 2011253085
(43) Date of publication of application: 24.09.2014
(73) Proprietor: Nitto Denko Corporation, Ibaraki-shi, Osaka 567-8680 (JP)
(72) Inventor: AYABE, Tokiyoshi, Sapporo-shi Hokkaido 001-0018 (JP); NAKAMURA, Kiminori, Sapporo-shi Hokkaido 001-0018 (JP); MINOMI, Kenjiro, Ibaraki-shi Osaka 567-8680 (JP); TANAKA, Yasunobu, Ibaraki-shi Osaka 567-8680 (JP)
(74) Representative: Hoffmann Eitle
(86) International application number: PCT/JP2012/079783
(87) International publication number: WO 2013/073667

(56) References cited:
- EP-A1- 1 842 557
- WO-A1-2006/068232
- WO-A1-2010/014117
- WO-A1-2010/014117
- WO-A2-2012/170957
- JP-A- 2009 221 164
- JP-A- 2010 059 124
- JP-A- 2010 539 245
- M Kidd ET AL: "Online Submissions: wjg CTGF, intestinal stellate cells and carcinoid fibrogenesis INTRODUCTION", World J Gastroenterol October, 1 January 2007 (2007-01-01), XP055160575, Retrieved from the Internet: URL:http://www.ncbi.nlm.nih.gov/pmc/articl es/PMC4171302/pdf/WJG-13-5208.pdf [retrieved on 2015-01-08]
- SAWAKO YOSHIOKA ET AL: "Isolation of intermediate stellate cells in the mouse small intestine", DAI 33 KAI ANNUAL MEETING OF THE MOLECULAR BIOLOGY SOCIETY OF JAPAN,, 10 December 2010 (2010-12-10), pages 4P-1143, XP008172637,
- SAWAKO YOSHIOKA ET AL: "Identification of stellate cells in the mouse small intestine", DAI 32 KAI ANNUAL MEETING OF THE MOLECULAR BIOLOGY SOCIETY OF JAPAN,, vol. 32nd, no. 3, 11 December 2009 (2009-12-11), page 240, XP008172820,
- NAGANUMA H ET AL.: "Shocho Kanmaku yori Hassei shi, Shinkeikei eno Bunka o Shimeshita Akusei Ichokangai Kanshitsu Shuyo no Ichirei", SENDAI SHIRITSU BYOIN IGAKU ZASSHI, vol. 27, 25 June 2007 (2007-06-25), pages 51-57, XP008172822,
- RIEDER F & FIOCCHI C: "Intestinal fibrosis in inflammatory bowel disease - Current knowledge and future perspectives", JOURNAL OF CROHN'S AND COLITIS, vol. 2, no. 4, 1 December 2008 (2008-12-01), pages 279-290, XP025692409, ISSN: 1873-9946, DOI: 10.1016/J.CROHNS.2008.05.009 [retrieved on 2008-07-03]
- VAN ROSSEN E ET AL: "Vinculin and cellular retinol-binding protein-1 are markers for quiescent and activated hepatic stellate cells in formalin-fixed paraffin embedded human liver", HISTOCHEMISTRY AND CELL BIOLOGY, vol. 131, no. 3, 4 December 2008 (2008-12-04), pages 313-325, XP019714781, ISSN: 1432-119X
- CASTILHO-FERNANDES A ET AL: "Human hepatic stellate cell line (LX-2) exhibits characteristics of bone marrow-derived mesenchymal stem cells", EXPERIMENTAL AND MOLECULAR PATHOLOGY, vol. 91, no. 3, November 2011 (2011-11), pages 664-672, XP028111727, ISSN: 0014-4800, DOI: 10.1016/J.YEXMP.2011.09.002 [retrieved on 2011-09-09]
- SPECA S ET AL: "Cellular and molecular mechanisms of intestinal fibrosis", WORLD JOURNAL OF GASTROENTEROLOGY, vol. 18, no. 28, 28 July 2012 (2012-07-28) , pages 3635-3661, XP55181001, ISSN: 1007-9327, DOI: 10.3748/wjg.v18.i28.3635
- YOSHIRO NIITSU: 'Kansei Saibo o Hyoteki to shita Kankohen no Chiryo Senryaku' EXPERIMENTAL MEDICINE vol. 29, no. 13, August 2011, pages 2114 - 2122, XP008172815
- YOSHIRO NIITSU: 'Treatment of Organ Fibrosis by siRNAHSP47' IBN SEMINAR SERIES: TREATMENT OF ORGAN FIBROSIS BY SIRNAHSP47 2010, JAPAN, XP055146428 Retrieved from the Internet: <URL:http:// www.ibn.a-star.edu.sg/media centre 8.php? expandable=3&eventid=306>
- YOSHIRO NIITSU: 'Zoki Sen'isho Chiryo no Shintenkai' THE JOURNAL OF THE JAPANESE RESPIRATORY SOCIETY 1343-3490 vol. 47, no. SUPPL., 2009, page 67, XP008172636
- KIDD, M. ET AL.: 'CTGF, intestinal stellate cells and carcinoid fibrogenesis' WORLD J GASTROENTEROL vol. 13, no. 39, 2007, pages 5208 - 16, XP055144306
- HIROSHI NAGANUMA ET AL.: 'Shocho Kanmaku yori Hassei shi, Shinkeikei eno Bunka o Shimeshita Akusei Ichokangai Kanshitsu Shuyo no Ichirei' SENDAI SHIRITSU BYOIN IGAKU ZASSHI vol. 27, 25 June 2007, pages 51 - 57, XP008172822 Retrieved from the Internet: <URL:http://www.hospital. city.sendai.jp/pdf/pdf-gan09.pdf>
- KOJI KUBOTA ET AL.: 'A Case of Gastrointestinal Stromal Tumor of the Mesentery' JAPANESE JOURNAL OF GASTROENTEROLOGICAL SURGERY vol. 41, no. 4, 01 April 2008, pages 435 - 440, XP055144318 Retrieved from the Internet: <URL:http:// journal.jsgs.or.jp/pdf/041040435.pdf>
- SAWAKO YOSHIOKA ET AL.: 'Identification of stellate cells in the mouse small intestine' DAI 32 KAI ANNUAL MEETING OF THE MOLECULAR BIOLOGY SOCIETY OF JAPAN 11 December 2009, page 240, XP008172820
- SAWAKO YOSHIOKA ET AL.: 'Isolation of intermediate stellate cells in the mouse small intestine' DAI 33 KAI ANNUAL MEETING OF THE MOLECULAR BIOLOGY SOCIETY OF JAPAN 10 December 2010, XP008172637
- J. HENRI BAYLE ET AL: "Rapamycin Analogs with Differential Binding Specificity Permit Orthogonal Control of Protein Activity", CHEMISTRY AND BIOLOGY., vol. 13, no. 1, 1 January 2006 (2006-01-01), pages 99-107, XP055469985, GB ISSN: 1074-5521, DOI: 10.1016/j.chembiol.2005.10.017
- SARASTE ANTTI ET AL: "Morphologic and biochemical hallmarks of apoptosis", CARDIOVASCULAR RESEARCH, vol. 45, no. 3, February 2000 (2000-02), pages 528-537, ISSN: 0008-6363

### SEQUENCE LISTING

<110> NITTO DENKO CORPORATION
<120> AGENT FOR TREATING FIBROSIS OF THE INTESTINE
<130> PCT2547ND
<150> JP2011-253085
   <151> 2011-11-18
<160> 16
<170> PatentIn version 3.5
<210> 1
   <211> 18
   <212> DNA
   <213> Artificial sequence
<220>
   <223> Forward primer for vimentin
<400> 1
   tgcgccagca gtatgaaa 18
<210> 2
   <211> 20
   <212> DNA
   <213> Artificial sequence
<220>
   <223> Reverse primer for vimentin
<400> 2
   gcctcagaga ggtcagcaaa 20
<210> 3
   <211> 19
   <212> DNA
   <213> Artificial sequence
<220>
   <223> Forward primer for alpha-SMA
<400> 3
   tcaccattgg aaacgaacg 19
<210> 4
   <211> 20
   <212> DNA
   <213> Artificial sequence
<220>
   <223> Reverse primer for alpha-SMA
<400> 4
   ataggtggtt tcgtggatgc 20
<210> 5
   <211> 20
   <212> DNA
   <213> Artificial sequence
<220>
   <223> Forward primer for ADRP
<400> 5
   cctcagctct cctgttaggc 20
<210> 6
   <211> 20
   <212> DNA
   <213> Artificial sequence
<220>
   <223> Reverse primer for ADRP
<400> 6
   cactactgct gctgccattt 20
<210> 7
   <211> 20
   <212> DNA
   <213> Artificial sequence
<220>
   <223> Forward primer for LRAT
<400> 7
   gaaggtggtc tccaacaagc 20
<210> 8
   <211> 20
   <212> DNA
   <213> Artificial sequence
<220>
   <223> Reverse primer for LRAT
<400> 8
   tactgtgtcc acacggatgc 20
<210> 9
   <211> 20
   <212> DNA
   <213> Artificial sequence
<220>
   <223> Forward primer for LXR beta
<400> 9
   gctctgccta catcgtggtc 20
<210> 10
   <211> 18
   <212> DNA
   <213> Artificial sequence
<220>
   <223> Reverse primer for LXR beta
<400> 10
   ctcatggccc agcatctt 18
<210> 11
   <211> 19
   <212> RNA
   <213> Artificial sequence
<220>
   <223> Mouse HSP47 siRNA sense strand
<400> 11
   ggacaggccu guacaacua 19
<210> 12
   <211> 19
   <212> RNA
   <213> Artificial sequence
<220>
   <223> Mouse HSP47 siRNA antisense strand
<400> 12
   uaguuguaca ggccugucc 19
<210> 13
   <211> 19
   <212> RNA
   <213> Artificial sequence
<220>
   <223> Random siRNA sense strand
<400> 13
   ccuccaaacc aauuggagg 19
<210> 14
   <211> 19
   <212> RNA
   <213> Artificial sequence
<220>
   <223> Random siRNA antisense strand
<400> 14
   ccuccaauug guuuggagg 19
<210> 15
   <211> 18
   <212> DNA
   <213> Artificial sequence
<220>
   <223> Forward primer for mouse HSP47
<400> 15
   gaaggctgtc gccatctc 18
<210> 16
   <211> 18
   <212> DNA
   <213> Artificial sequence
<220>
   <223> Reverse primer for mouse HSP47
<400> 16
   cccagtcctg ccagatgt 18

## Description

### [Technical Field]

The present invention relates to a substance delivery carrier targeted to extracellular matrix-producing cells in the intestine, and a composition for treating fibrosis of the intestine as defined in the claims.

### [Background Art]

Fibrosis of the intestine is a pathological condition characterized by excessive deposition of scar tissue on the wall of the intestine and follows chronic inflammation of the intestine, such as for example a chronic inflammatory bowel disease (IBD) or tissue injury due to radiation (Non-Patent Literature 1). Inflammatory bowel diseases include Crohn's disease and ulcerative colitis; for example, in Crohn's disease fibrosis of the intestine occurs in about 25% to 30% of patients. Fibrosis of the intestine forms a stricture of the intestine when it has progressed, makes it difficult for food to pass through, and becomes an important cause of impairment of the QOL of a diseased subject. However, the mechanism of fibrosis of the intestine has not yet been clarified, and because of this no definitive therapy is currently established.

Conventional treatment for fibrosis of the intestine is focused on treatment of the causative inflammation; various anti-inflammatory agents, for example, an aminosalicylic acid-based drug such as sulfasalazine, mesalamine, alsalazine, or balsalazide, a corticosteroid drug such as prednisolone or budesonide, an immunosuppressive agent such as azathioprine, mercaptopurine, cyclosporin, or methotrexate, a TNFα inhibitor such as infliximab and, furthermore, an antibiotic such as metronidazole or Ciproxan are used. However, these anti-inflammatory agents do not directly treat the fibrosis, and in serious fibrosis of the intestine it becomes necessary to surgically remove fibrotic tissue, which would impose an enormous burden on the patient.

In light of such circumstances, several attempts to directly treat fibrosis of the intestine have been made in recent years. As a result, it has been reported that a medicinal agent such as, for example, a TGFβ1 vaccine (Non-Patent Literature 2), pentoxifylline or a metabolite thereof (Non-Patent Literature 3), a phosphodiesterase 4 inhibitor (Non-Patent Literature 4), an HMG-CoA reductase inhibitor (Non-Patent Literature 5), daikenchuto (Non-Patent Literature 6), pravastatin (Non-Patent Literature 7), a lipoxin A₄ analog (Patent Literature 1), or a sulfate group transferase inhibitor (Patent Literature 2) has exhibited a certain degree of success in a fibrosis of the intestine model animal, etc. However, none of these medicinal agents are satisfactory, and further development of agents for treating fibrosis of the intestine is needed.

Rieder and Fiocchi (i.e. Journal of Crohn's and Colitis (2008) 2, 279-290 concerns a review of intestinal fibrosis in inflammatory bowel disease. This review article explains on page 282 that in contrast to the wealth of data on the role of stellate cells in liver and pancreatic fibrosis, very limited information is available on intestinal stellate cells.

### [Citation List]

### [Patent Literature]

[Patent Literature 1] WO 2008/022807
[Patent Literature 2] WO 2009/084232
[Patent Literature 3] WO 2006/068232
[Patent Literature 4] WO 2009/036368
[Patent Literature 5] WO 2010/014117
[Patent Literature 6] WO 2009/116257
[Patent Literature 7] WO 2010/026766

### [Non-Patent Literature]

[Non-Patent Literature 1] Rieder and Fiocchi, Nat Rev Gastroenterol Hepatol. 2009 Apr; 6 (4): 228-35
[Non-Patent Literature 2] Ma et al., Inflamm Bowel Dis. 2010 Jun; 16 (6): 1040-50
[Non-Patent Literature 3] Peterson et al., Eur J Pharmacol. 2011 Jul 15; 662 (1-3): 47-54
[Non-Patent Literature 4] Videla et al., J Pharmacol Exp Ther. 2006 Feb; 316 (2): 940-5
[Non-Patent Literature 5] http://www.ncbi.nlm.nih.gov/pubmed/21909991
[Non-Patent Literature 6] Inoue et al., Biol Pharm Bull. 2011; 34 (11): 1659-65
[Non-Patent Literature 7] Haydont et al., Clin Cancer Res. 2007 Sep 15; 13 (18 Pt 1): 5331-40

### [Summary of Invention]

### [Technical Problem]

The current invention is defined, *inter alia,* by the following items:
1. A pharmaceutical composition for use in treating fibrosis of the intestine, the composition comprising a carrier comprising a retinoid which promotes the delivery of the composition to extracellular matrix-producing cells in the intestine and a drug for controlling the activity or growth of extracellular matrix-producing cells in the intestine, wherein the fibrosis of the intestine is Crohn's disease or ulcerative colitis, and wherein the drug for controlling the activity or growth of extracellular matrix-producing cells in the intestine is HSP47 siRNA.
2. A pharmaceutical composition for use in recovering tissue of the intestine that has been altered by fibrosis to at least a state with a lesser degree of fibrosis, the composition comprising a carrier comprising a retinoid which promotes the delivery of the composition to extracellular matrix-producing cells in the intestine and a drug for controlling the activity or growth of extracellular matrix-producing cells in the intestine, wherein the fibrosis is Crohn's disease or ulcerative colitis, and wherein the drug for controlling the activity or growth of extracellular matrix-producing cells in the intestine is HSP47 siRNA.
3. The pharmaceutical composition for use according to item 1 or 2, wherein the retinoid comprises retinol.
4. The pharmaceutical composition for use according to any one of items 1 to 3, wherein the carrier comprises a retinoid and a carrier component other than the retinoid, the molar ratio of the retinoid to the carrier component other than the retinoid being 8:1 to 1:4.
5. The pharmaceutical composition for use according to any one of items 1 to 4, wherein it is in a form prepared at the time of use.
6. A kit for preparing the pharmaceutical composition according to any one of items 1 to 5, the kit comprising one or more containers that comprise either singly or in combination the drug for controlling the activity or growth of extracellular matrix-producing cells in the intestine, the retinoid and, as necessary, a carrier constituent other than the retinoid, wherein the drug for controlling the activity or growth of extracellular matrix-producing cells in the intestine is HSP47 siRNA.
7. A process for producing the pharmaceutical composition for use in treating fibrosis of the intestine according to any one of items 1 and 3 to 5 or a pharmaceutical composition for use in regenerating normal tissue of the intestine from fibrotic tissue of the intestine according to any one of items 2 to 5, the process comprising a step of formulating a retinoid which promotes the delivery of the composition to extracellular matrix-producing cells in the intestine, and a drug for controlling the activity or growth of extracellular matrix-producing cells in the intestine as an active ingredient, wherein the drug for controlling the activity or growth of extracellular matrix-producing cells in the intestine is HSP47 siRNA.

It is an object of the present description to provide a carrier that can deliver a substance such as a drug specifically to extracellular matrix-producing cells in the intestine, and a fibrosis of the intestine treatment agent and method for treating fibrosis of the intestine utilizing said carrier.

### [Solution to Problem]

The present inventors have succeeded in isolating extracellular matrix-producing cells from fibrotic tissue of the intestine during an investigation into a novel agent for treating fibrosis of the intestine and, furthermore, have found that a carrier that includes a retinoid as a targeting agent delivers an extracellular matrix production inhibitor to said cells with high efficiency and markedly inhibits expression of a molecule involved in extracellular matrix production.

It is known that a carrier that includes vitamin A can deliver a drug to hepatic stellate cells (Patent Literature 3, Patent Literature 4) or a hepatic stellate cell line (Patent Literature 3, Patent Literature 5), or extracellular matrix-producing cells in the lung (Patent Literature 6) and the bone marrow (Patent Literature 7) and that a composition in which siRNA for HSP47 is carried on the above carrier can improve hepatic fibrosis (Patent Literature 3), pulmonary fibrosis (Patent Literature 6), and myelofibrosis (Patent Literature 7), but any relationship to extracellular matrix-producing cells in the intestine or fibrosis of the intestine is so far completely unknown.

That is, the present description relates to the following.
(1) A carrier for delivering a substance to extracellular matrix-producing cells in the intestine, the carrier comprising a retinoid as a targeting agent for extracellular matrix-producing cells in the intestine.
(2) The carrier according to (1) above, wherein the retinoid includes retinol.
(3) The carrier according to (1) or (2) above, wherein it includes a retinoid and a carrier component other than the retinoid, the molar ratio of the retinoid to the carrier component other than the retinoid being 8:1 to 1:4.
(4) A pharmaceutical composition for treating fibrosis of the intestine, the composition comprising the carrier according to any one of (1) to (3) above and a drug for controlling the activity or growth of extracellular matrix-producing cells in the intestine.
(5) A pharmaceutical composition for regenerating normal tissue of the intestine from fibrotic tissue of the intestine, the composition comprising the carrier according to any one of (1) to (3) above and a drug for controlling the activity or growth of extracellular matrix-producing cells in the intestine.
(6) The pharmaceutical composition according to (4) or (5) above, wherein the drug for controlling the activity or growth of extracellular matrix-producing cells in the intestine is selected from the group consisting of a substance for inhibiting the production and secretion of an extracellular matrix component, a cell growth inhibitor, an apoptosis-inducing substance, a TIMP inhibitor, and an α1-antitrypsin inhibitor.
(7) The pharmaceutical composition according to (6) above, wherein the substance for inhibiting the production and secretion of an extracellular matrix component is an HSP47 inhibitor.
(8) The pharmaceutical composition according to any one of (4) to (7) above, wherein it is in a form prepared at the time of use.
(9) A kit for preparing the pharmaceutical composition according to any one of (4) to (8) above, the kit comprising one or more containers that comprise either singly or in combination the drug for controlling the activity or growth of extracellular matrix-producing cells in the intestine, the retinoid and, as necessary, a carrier constituent other than the retinoid.
(10) A process for producing a carrier for delivering a substance to extracellular matrix-producing cells in the intestine, the process comprising a step of formulating a retinoid as a targeting agent for extracellular matrix-producing cells in the intestine.
(11) A process for producing a pharmaceutical composition for treating fibrosis of the intestine or a pharmaceutical composition for regenerating normal tissue of the intestine from fibrotic tissue of the intestine, the process comprising a step of formulating a retinoid as a targeting agent for extracellular matrix-producing cells in the intestine, and a drug for controlling the activity or growth of extracellular matrix-producing cells in the intestine as an active ingredient.
(12) An extracellular matrix-producing cell line of the intestine, the cell line being derived from fibrotic bowel tissue harvested from a subject suffering from fibrosis of the intestine and having a vimentin-positive, aSMA-negative, and GFAP-negative phenotype.
(13) A method for isolating extracellular matrix-producing cells of the intestine, the method comprising
   (i) a step of obtaining cells from fibrotic bowel tissue harvested from a subject suffering from fibrosis of the intestine, and
   (ii) a step of selecting cells having a vimentin-positive, aSMA-negative, and GFAP-negative phenotype from the cells obtained in (i) .
(14) A method for preparing an extracellular matrix-producing cell line of the intestine, the method comprising
   (i) a step of obtaining cells from fibrotic bowel tissue harvested from a subject suffering from fibrosis of the intestine, and
   (ii) a step of selecting cells having a vimentin-positive, aSMA-negative, and GFAP-negative phenotype from the cells obtained in (i),
   the method also comprising a step of immortalizing cells subsequent to step (i) or (ii).
(15) A method for screening a factor for treating fibrosis of the intestine, the method comprising
   (i) a step of making the cell line according to (12) above to coexist with a test factor, and
   (ii) a step of detecting a change in the cell line due to coexistence with the test factor.
(16) A kit for screening a factor for treating fibrosis of the intestine, the kit comprising the cell line according to (12) above.

### [Advantageous Effects of Invention]

While the exact mode of action of the composition for treating fibrosis of the intestine of the present description has not yet been completely clarified, it is believed that the retinoid functions as an agent that targets extracellular matrix-producing cells in the intestine, and delivers an active ingredient such as for example a drug that controls the activity or growth of extracellular matrix-producing cells in the intestine to such cells, thereby exhibiting an effect against fibrosis of the intestine.

Therefore, since an active ingredient can be efficiently delivered to the site of action and, further, to target cells, by using the carrier of the present description comprising a retinoid as a targeting agent, the cure, suppression of progression, and prevention of onset of fibrosis of the intestine, for which there has been no definitive therapeutic method to date, are made possible, and the present carrier thus contributes significantly to human medicine and veterinary medicine.

Moreover, the carrier of the present description can be combined with any medicinal agent (for example, an existing therapeutic drug for fibrosis of the intestine) to increase its efficiency of action; there is therefore also the advantage that there are a wide range of applications in terms of formulation, enabling the production of effective therapeutic agents to be facilitated.

Furthermore, the cell line of the present description can be utilized in screening of a drug for treating fibrosis of the intestine or in elucidation of the mechanism of fibrosis of the intestine, and contributes to the development of new treatment agents or treatment methods for fibrosis of the intestine.

### [Brief Description of Drawings]

FIG. 1 is a photographic diagram showing localization of stellate cell-like mesenchymal cells in a site of fibrosis of the intestine in a SAMP1/Yit mouse. It shows an image of azan staining (a), and images of immunofluorescence staining by means of an anti-vimentin antibody (b), an anti-αSMA antibody (c), and an anti-GFAP antibody (d). The arrows show the location of stellate cell-like mesenchymal cells. The scale bar denotes 100 *µ*m for (a) and 50 *µ*m for (b) to (d).
FIG. 2 is a photographic diagram showing expression of vimentin and αSMA in mesenchymal cell line IC10_F2 isolated from a site of fibrosis of the intestine in a SAMP1/Yit mouse (top) and vimentin-positive, αSMA-negative, GFAP-negative cell line IC10_F2_E9 derived therefrom (bottom). The scale bar denotes 50 *µ*m.
FIG. 3 is a graph showing relative expression of vimentin, αSMA, ADRP, LRAT, and LXRβ in IC10_F2 cells and IC10_F2_E9 cells.
FIG. 4 is a graph showing relative expression of HSP47 when siRNA for HSP47 was introduced into IC10_F2_E9 cells by being carried by a VA-coupled liposome.

### [Description of Embodiments]

One aspect of the present description relates to a carrier for delivering a substance to extracellular matrix-producing cells in the intestine, the carrier comprising a retinoid as a targeting agent for extracellular matrix-producing cells in the intestine. One embodiment of the carrier of the present description includes an effective amount of a retinoid for targeting extracellular matrix-producing cells in the intestine. Furthermore, one embodiment of the carrier of the present description relates to a carrier targeted to extracellular matrix-producing cells in the intestine by means of a retinoid.

In the present description, the extracellular matrix-producing cells in the intestine are not particularly limited as long as they are cells that are present in the intestine and have the ability to produce extracellular matrix; examples thereof include stellate cell-like cells, fibroblasts, pericytes, fibrocytes, and myofibroblasts that are present in the intestine. The matrix-producing cells that are present in the intestine can include not only those derived from cells that are present in the intestine but also those derived from fibrocytes in circulating blood and those transformed from epithelial cells or endothelial cells by endothelial-mesenchymal transdifferentiation (Non-Patent Literature 1).

Examples of the stellate cell-like cells include cells having a vimentin-positive, αSMA (α smooth muscle actin)-negative, and GFAP (glial fibrillary acidic protein)-negative phenotype that have been identified in the examples below. Such cells are identified by immunostaining using an anti-vimentin antibody, anti-aSMA antibody, and anti-GFAP antibody that are detectably labeled. The cells may express a VA (vitamin A) storage-related gene, for example, ADRP (adipose differentiation-related protein), LRAT (lecithin retinol acyl transferase), and/or LXRβ (liver X receptor β), etc. Hepatic stellate cells are activated when cultured in vitro and become αSMA-positive and GFAP-positive, but the stellate cell-like cells do not become αSMA- and GFAP-positive even when cultured in vitro. Myofibroblasts are characterized by expressing vimentin and αSMA; fibroblasts express vimentin characteristic of mesenchymal cells but do not express αSMA, and they can be identified by double staining, etc. of vimentin and αSMA. Extracellular matrix-producing cells in the intestine can also be obtained by selecting, from cells obtained from tissue of the intestine, those having a vimentin-positive, aSMA-negative, and GFAP-negative phenotype.

The retinoid of the present description functions as a targeting agent for extracellular matrix-producing cells in the intestine, and promotes the specific delivery of a substance to these cells. The mechanism of the promotion of substance delivery by the retinoid has not yet been completely clarified; however, for example, it is thought that a retinoid that has specifically bound to a retinol binding protein (RBP) is taken into an extracellular matrix-producing cell in the intestine through a certain receptor present on the surface of this cell.

A retinoid is a member of a class of compounds having a skeleton in which four isoprenoid units are joined in a head-to-tail manner (see G. P. Moss, 'Biochemical Nomenclature and Related Documents', 2nd Ed. Portland Press, pp. 247-251 (1992)). Vitamin A is a generic descriptor for a retinoid exhibiting qualitatively the biological activity of retinol. The retinoid that can be used in the present invention is not particularly limited, and examples thereof include retinol (including all-trans-retinol), retinal, retinoic acid (including tretinoin), retinoid derivatives such as an ester of retinol and a fatty acid, an ester of an aliphatic alcohol and retinoic acid, etretinate, isotretinoin, adapalene, acitretine, tazarotene, and retinyl palmitate, and vitamin A analogues such as fenretinide (4-HPR) and bexarotene.

Of these, retinol, retinal, retinoic acid, an ester of retinol and a fatty acid (such as for example retinyl acetate, retinyl palmitate, retinyl stearate, and retinyl laurate) and an ester of an aliphatic alcohol and retinoic acid (such as for example ethyl retinoate) are preferable from the viewpoint of efficiency of specific delivery of a substance to extracellular matrix-producing cells in the intestine.

All retinoid isomers including cis-trans isomers are included in the scope of the present description. The retinoid may be substituted with one or more substituents. The retinoid in the present description includes a retinoid in an isolated form as well as in the form of a solution or mixture with a medium that can dissolve or retain the retinoid.

The retinoid in the present description includes a compound containing a retinoid as a part thereof (retinoid moiety-containing compound). Such a compound may contain one or more retinoid moieties, for example, one, two, three, four, five, six, seven, eight, nine, ten, or more moieties. In such a compound, a retinoid may be present in a state in which its RBP binding site (e.g. a cyclohexene ring moiety in the case of retinol) can bind to an RBP. Examples of such a compound include, but are not limited to, one in which one or more retinoids and PEG or a derivative thereof are bonded. In such a retinoid-PEG conjugate, an RBP non-binding site (e.g. a moiety other than a cyclohexene ring moiety in the case of retinol, for example, the OH group, etc.) of a retinoid may be covalently bonded to PEG or a derivative thereof. The PEG or a derivative thereof may have 1 to 50 repeating units (CH₂CH₂O). The PEG or a derivative thereof may have a molecular weight of 200 to 4000 g/mol. The PEG or a derivative thereof may be linear or branched. The PEG derivative may have a group suitable for bonding to a retinoid at a terminal, for example, an amino group, etc. The PEG derivative may have one or more amide groups in the chain, for example, one, two, three, four, five, six, seven, eight, nine, ten, or more amide groups.

The carrier of the present description may be constituted from the retinoid on its own or may be constituted by binding the retinoid to a carrier constituent other than the retinoid, or by enclosing it therein. Therefore, the carrier of the present description may include a carrier constituent other than the retinoid. Such a component is not particularly limited, and any component known in the medicinal and pharmaceutical fields may be used, but those that can enclose the retinoid or can bind to the retinoid are preferable.

Examples of such components include a lipid, for example, a phospholipid such as glycerophospholipid, a sphingolipid such as sphingomyelin, a sterol such as cholesterol, a vegetable oil such as soybean oil or poppy seed oil, a mineral oil, or a lecithin such as egg-yolk lecithin, and a polymer, but the examples are not limited thereto. Among them, those that can form a liposome, for example, a natural phospholipid such as lecithin, a semisynthetic phospholipid such as dimyristoylphosphatidylcholine (DMPC), dipalmitoylphosphatidylcholine (DPPC), or distearoylphosphatidylcholine (DSPC), dioleylphosphatidylethanolamine (DOPE), dilauroylphosphatidylcholine (DLPC), cholesterol, etc. are preferable.

A particularly preferred component is a component that can avoid capture by the reticuloendothelial system, examples thereof including cationic lipids such as N-(α-trimethylammonioacetyl)-didodecyl-D-glutamate chloride (TMAG), *N,N',N'',N'''-*tetramethyl*-N,N',N'',N'''-*tetrapalmitylspermine (TMTPS), 2,3-dioleyloxy-*N*-[2-(sperminecarboxamido)ethyl]-*N,N*-dimethyl-1 -propanaminium trifluoroacetate (DOSPA), *N*-[1-(2,3-dioleyloxy)propyl]-*N,N,N*-trimethylammonium chloride (DOTMA), dioctadecyldimethylammonium chloride (DODAC), didodecylammonium bromide (DDAB), 1,2-dioleyloxy-3-trimethylammoniopropane (DOTAP), 3β-[*N-*(*N',N'*-dimethylaminoethane)carbamoyl]cholesterol (DC-Chol), 1,2-dimyristoyloxypropyl-3-dimethylhydroxyethylammonium bromide (DMRIE), and o,o'-ditetradecanoyl-*N*-(α-trimethylammonioacetyl)diethanolamin e chloride (DC-6-14).

The carrier in the present description may have a specific three-dimensional structure. Such a structure is not limited, and examples thereof include a straight-chain or branched linear structure, a filmm structure, and a spherical structure. Therefore, the carrier may have, without limitation, any three-dimensional form such as a dendrimer, a dendron, a micelle, a liposome, an emulsion, a microsphere, or a nanosphere. Furthermore, one embodiment of the carrier in the present description is a carrier that enables active targeting by means of a targeting agent (including a targeting molecule, a targeting moiety, etc.). A carrier that has a three-dimensional configuration or a carrier that enables active targeting is well known in the present technical field (ref. e.g. Marcucci and Lefoulon, Drug Discov Today. 2004 Mar 1; 9 (5): 219-28, Torchilin, Eur J Pharm Sci. 2000 Oct; 11 Suppl 2: S81-91, etc.).

Binding of the retinoid to the carrier of the present description or the enclosing of it therein is also made possible by binding the retinoid to or enclosing it in a carrier constituent other than the retinoid by a chemical and/or physical method. Alternatively, the retinoid can be bound to or enclosed in the carrier of the present description by mixing the retinoid and the carrier constituent other than the retinoid during the preparation of the carrier. The amount of the retinoid in the carrier of the present description may be for example 0.01 to 1000 nmol/µL, preferably 0.1 to 100 nmol/µL. Furthermore, in the carrier of the present description containing a retinoid and a carrier constituent other than a retinoid, the molar ratio of the retinoid to the carrier constituent other than the retinoid is not limited and may be for example 8:1 to 1:4, or 4:1 to 1:2. The retinoid may be bound to or enclosed in the carrier before loading a substance to be delivered to the carrier; or the carrier, retinoid, and a substance to be delivered may be mixed simultaneously; or the retinoid may be admixed with the carrier already carrying the substance to be delivered, etc. Therefore, the present description also relates to a process for producing a formulation specific to extracellular matrix-producing cells in the intestine, the process comprising a step of binding a retinoid to any existing drug-coupled carrier or drug-encapsulated carrier, for example, a liposomal formulation such as DaunoXome®, Doxil, Caelyx®, or Myocet®.

The carrier of the present description may be in any form as long as a desired substance or object can be transported to target extracellular matrix-producing cells in the intestine, and examples thereof include, but are not limited to, a polymer, a dendrimer, a dendron, a macromolecular micelle, a liposome, an emulsion, microspheres, and nanospheres. In the present description, a liposomal form is preferable among these from the viewpoint of high delivery efficiency, wide selection of substances to be delivered, and ease of formulation, etc., and a cationic liposome containing a cationic lipid is particularly preferable. In the case where the carrier is in the form of a liposome, the molar ratio of the retinoid to other constituent lipids of the liposome is preferably 8:1 to 1:4, and more preferably 4:1 to 1:2, from the viewpoint of the efficiency of binding the retinoid to the carrier or enclosing it therein.

The carrier of the present description may contain a substance to be transported within its interior, it may be attached to the exterior of a substance to be transported, or it may be mixed with a substance to be transported, as long as it contains a retinoid in a form such that the retinoid is able to function as a targeting agent. 'Function as a targeting agent' herein means that the carrier that includes a retinoid reaches and/or is taken up by the target cells, i.e., extracellular matrix-producing cells in the intestine, more rapidly and/or in a larger quantity than with a carrier not comprising the retinoid, and this may easily be confirmed by, for example, adding a labeled or label-containing carrier to a culture of target cells and analyzing the distribution of the label after a predetermined period of time. Structurally, this requirement can be satisfied, for example, if a retinoid is at least partially exposed to the exterior of the carrier (for example, when the carrier has a three-dimensional structure, etc.) or the formulation containing the carrier, at the latest by the time it reaches the target cells. The 'formulation' referred to here is a concept that includes the composition of the present invention, which is described later, and that further has a form. Whether or not the retinoid is exposed at the exterior of a formulation can be evaluated by contacting the formulation with a substance that specifically binds to a retinoid, such as for example a retinol binding protein (RBP), and examining its binding to the formulation.

Exposing a retinoid at least partially to the exterior of the carrier or the formulation at the latest by the time it reaches the target cells may be achieved for example by adjusting the compounding ratio of the retinoid and carrier constituents other than the retinoid. Furthermore, when the carrier has the form of a lipid structure such as a liposome, when for example forming a complex from a retinoid and a carrier constituent other than the retinoid, a method may be used in which first a lipid structure formed from the carrier constituent other than the retinoid is diluted in an aqueous solution, and this is then contacted and mixed, etc. with the retinoid. In this case, the retinoid may be in a state in which it is dissolved in a solvent, for example, an organic solvent such as DMSO. The lipid structure referred to here means a structure containing a lipid as a constituent and having any three-dimensional structure, for example, a shape such as a linear form, a film form, or a spherical form, and examples thereof include, but are not limited to, a liposome, a micelle, a lipid microsphere, a lipid nanosphere, and a lipid emulsion. The ability to apply to another drug carrier the same targeting agent as one targeting a liposome is described in for example Zhao and Lee, Adv Drug Deliv Rev. 2004; 56(8): 1193-204, Temming et al., Drug Resist Updat. 2005; 8(6): 381-402, etc.

The lipid structure may be stabilized by for example adjusting the osmotic pressure by the use of an osmotic pressure-adjusting agent such as a salt, a saccharide such as sucrose, glucose, or maltose, or a polyhydric alcohol such as glycerol or propylene glycol, and preferably sucrose or glucose. Furthermore, the pH may be adjusted by adding an appropriate amount of a pH adjusting agent such as a salt or a buffer. It is therefore possible to carry out production, storage, etc. of a lipid structure in a medium containing the above substances. In this case, the concentration of the osmotic pressure-adjusting agent is preferably adjusted so as to be isotonic with blood. For example, in the case of sucrose the concentration thereof in a medium is not limited but may be 3 to 15 wt%, preferably 5 to 12 wt%, more preferably 8 to 10 wt%, and particularly 9 wt%, and in the case of glucose the concentration thereof in a medium is not limited but may be 1 to 10 wt%, preferably 3 to 8 wt%, more preferably 4 to 6 wt%, and particularly 5 wt%.

The present description also relates to a process for producing a carrier for delivering a substance to extracellular matrix-producing cells in the intestine, the process comprising a step of formulating a retinoid as a targeting agent for extracellular matrix-producing cells in the intestine. The method of formulating the retinoid is not particularly limited as long as, in the carrier in which it is formulated, the retinoid can function as a targeting agent to extracellular matrix-producing cells in the intestine, and for example various methods described herein may be used. Therefore, formulation of the retinoid may be carried out by binding the retinoid to or enclosing it in another constituent of the carrier by a chemical and/or physical method or by mixing the retinoid with another carrier constituent when preparing the carrier. The formulation amount of retinoid, etc. is as described above with respect to the carrier of the present description.

The substance to be delivered by the present carrier described herein is not particularly limited, and it preferably has a size such that it can physically move within the body of an organism from the site of administration to the site of a lesion where the target cells are present. Therefore, the carrier of the present description can transport not only a substance such as an atom, a molecule, a compound, a protein, or a nucleic acid, but also an object such as a vector, a virus particle, a cell, a drug-releasing system that includes one or more elements, or a micromachine. The substance to be delivered preferably has the property of exerting some effect on the target cells, and examples include those labeling the target cells or controlling (e.g. increasing or suppressing) the activity or growth of the target cells.

Therefore, in one instance of the present description, the substance to be delivered by the carrier includes 'a drug for controlling the activity or growth of extracellular matrix-producing cells in the intestine'. The activity of the extracellular matrix-producing cells in the intestine herein refers to various activities such as secretion, uptake, or migration exhibited by extracellular matrix-producing cells in the intestine, and in the present description, in particular, among these, it typically means an activity involved in the onset, progression, and/or recurrence of fibrosis of the intestine. Examples of such activity include, but are not limited to, the production/secretion of an extracellular matrix component such as collagen, proteoglycan, tenascin, fibronectin, thrombospondin, osteopontin, osteonectin, or elastin, and the suppression of decomposition activity of these extracellular matrix components.

Therefore, the drug for controlling the activity or growth of extracellular matrix-producing cells in the intestine referred to herein may be any drug that inhibits directly or indirectly the physical, chemical, and/or physiological action, etc. of these cells involved in the onset, progression, and/or recurrence of fibrosis of the intestine; examples include, but are not limited to, a substance for inhibiting the production and secretion of extracellular matrix components, etc., a cell growth inhibitor, an apoptosis-inducing substance, a TIMP (Tissue inhibitor of metalloproteinase) inhibitor, and an α1-antitrypsin inhibitor.

Examples of the substance for inhibiting the production and secretion of an extracellular matrix component, etc. include, but are not limited to, a substance such as an RNAi molecule, ribozyme, or antisense nucleic acid, or a substance having a dominant negative effect such as a dominant negative mutant, that inhibits expression of an extracellular matrix component such as collagen, proteoglycan, tenascin, fibronectin, thrombospondin, osteopontin, osteonectin, or elastin, a vector expressing same, and cells transformed thereby. Among the extracellular matrix components, drugs for inhibiting the production and secretion of collagen include, but are not limited to, inhibitors for HSP (Heat shock protein) 47, which is a collagen-specific molecular chaperone essential for intracellular transport and molecular maturation, which are common to the synthetic processes of various types of collagen, for example, an HSP47 expression inhibitor such as an RNAi molecule, ribozyme, or antisense nucleic acid for HSP47, a substance having a dominant negative effect such as a dominant negative mutant of HSP47, a vector expressing same, and cells transformed thereby.

Examples of the cell growth inhibitor include, but are not limited to, an alkylating agent such as ifosfamide, nimustine (e.g. nimustine hydrochloride), cyclophosphamide, dacarbazine, melphalan, or ranimustine, a metabolism antagonist such as gemcitabine (e.g. gemcitabine hydrochloride), enocitabine, cytarabine ocfosfate, a cytarabine preparation, tegafur/uracil, a tegafur/gimeracil/oteracil potassium combination drug (e.g. TS-1), doxifluridine, hydroxycarbamide, fluorouracil, methotrexate, or mercaptopurine, an antitumor antibiotic such as idarubicin (e.g. idarubicin hydrochloride), epirubicin (e.g. epirubicin hydrochloride), daunorubicin (e.g. daunorubicin hydrochloride, daunorubicin citrate), doxorubicin (e.g. doxorubicin hydrochloride), pirarubicin (e.g. pirarubicin hydrochloride), bleomycin (e.g. bleomycin hydrochloride), peplomycin (e.g. peplomycin sulfate), mitoxantrone (e.g. mitoxantrone hydrochloride), or mitomycin C, an alkaloid such as etoposide, irinotecan (e.g. irinotecan hydrochloride), vinorelbine (e.g. vinorelbine tartarate), docetaxel (e.g. docetaxel hydrate), paclitaxel, vincristine (e.g. vincristine sulfate), vindesine (e.g. vindesine sulfate), or vinblastine (e.g. vinblastine sulfate), a hormone therapy drug such as anastrozole, tamoxifen (e.g. tamoxifen citrate), toremifene (e.g. toremifene citrate), bicalutamide, flutamide, or estramustine (e.g. estramustine phosphate), a platinum complex such as carboplatin, cisplatin (CDDP), or nedaplatin, an angiogenesis inhibitor such as thalidomide, neovastat, or bevacizumab, and L-asparaginase.

Examples of the apoptosis-inducing substance include, but are not limited to, compound 861, gliotoxin, and atorvastatin.

Examples of the TIMP (e.g. TIMP1, TIMP2, TIMP3, etc.) inhibitor include, but are not limited to, a TIMP activity inhibitor such as an antibody for a TIMP, a TIMP production inhibitor such as an RNAi molecule, ribozyme, or antisense nucleic acid for a TIMP, a vector expressing same, and cells transformed thereby.

Examples of the α1-antitrypsin inhibitor include, but are not limited to, an α1-antitrypsin activity inhibitor such as an antibody for α1-antitrypsin, an α1-antitrypsin production inhibitor such as an RNAi molecule, ribozyme, or antisense nucleic acid for α1-antitrypsin, a vector expressing same, and cells transformed thereby.

Furthermore, the 'drug for controlling the activity or growth of extracellular matrix-producing cells in the intestine' in the present invention may be any drug that promotes directly or indirectly the physical, chemical, and/or physiological action, etc. of extracellular matrix-producing cells in the intestine involved directly or indirectly in inhibition of the onset, progression, and/or recurrence of fibrosis of the intestine.

The carrier of the present description may deliver one or more types of the above-mentioned drugs.

The RNAi molecule in the present description includes duplex RNAs such as siRNA (small interfering RNA), miRNA (micro RNA), shRNA (short hairpin RNA), piRNA (Piwi-interacting RNA), and rasiRNA (repeat associated siRNA) and modified forms thereof. An RNAi molecule and a vector expressing the RNAi molecule may be used for example in accordance with the teaching of a standard text (for example, Experimental Medicine Special Edition, Revised RNAi Experimental Protocol 2004, Yodosha, RNAi Experimental Frequently Asked Questions 2006, Yodosha, etc.).

Design of the RNAi molecule may be carried out appropriately by a person skilled in the art in accordance with the teaching of a standard text (Experimental Medicine Special Edition, Revised RNAi Experimental Protocol 2004, Yodosha, RNAi Experimental Frequently Asked Questions 2006, Yodosha) by reference to a messenger RNA sequence of a target gene and a known RNAi molecular sequence.

The nucleic acid in the present description includes RNA, DNA, PNA, or a complex thereof.

The substance to be delivered by the carrier of the present description also includes, but is not limited to, a drug, other than those described above, for inhibiting the onset, progression, and/or recurrence of fibrosis of the intestine, and examples include, but are not limited to, a TGFβ1 inhibitor (including a TGFβ1 vaccine), pentoxifylline and a metabolite thereof, a phosphodiesterase 4 inhibitor, an HMG-CoA reductase inhibitor, daikenchuto, pravastatin, a lipoxin A₄ analog, a sulfate group transferase inhibitor, an inhibitor for a fibrosis promoting factor (e.g. EGF, bFGF, FGF2, PDGF, IGF-I, IGF-II, CTGF, IL-1β, IL-4, IL-13, MCP-1, MIP-Iα, MIP-1β, MIP-3α, NOD1 ligand, TLR2, 4, and 5 ligands, galectin 3, hyaluronan, laminin, collagen, etc.), a drug for inhibiting inflammation that causes the onset of fibrosis of the intestine such as for example an aminosalicylic acid-based drug such as sulfasalazine, mesalamine, alsalazine, or balsalazide, a corticosteroid drug such as prednisolone or budesonide, an immunosuppressive agent such as azathioprine, mercaptopurine, cyclosporin, or methotrexate, a TNFα inhibitor such as infliximab and, moreover, an antibiotic such as metronidazole or Ciproxan. These drugs may be used in combination with the composition of the present description, which is described later. Here, 'being used in combination' includes administration of the composition of the present description and the drug at substantially the same time and administration of them with a time interval within the same treatment period. In the case of the former, the composition of the present description may be mixed with the drug and administered or they may be administered in succession without being mixed. In the case of the latter, the composition of the present description may be administered prior to or subsequent to the drug.

The drug belonging to the invention for controlling the activity or growth of extracellular matrix-producing cells in the intestine is an HSP47 inhibitor, namely, an siRNA for HSP47.

The substance or object delivered by the carrier of the present description may or may not be labeled. Labeling enables monitoring of the success or failure of delivery to target cells, or the increase and decrease of target cells, etc., and is particularly useful not only at the testing/research level but also at the clinical level. A label may be selected from any label known to a person skilled in the art such as, for example, any radioisotope, magnetic material, gas or substance that generates a gas under physiological conditions, element that exhibits nuclear magnetic resonance (e.g. hydrogen, phosphorus, sodium, fluorine, etc.), substance that affects the relaxation time of an element exhibiting nuclear magnetic resonance (e.g. a metal atom or compound containing same), substance that binds to a labeled substance (e.g. an antibody, etc.), fluorescent substance, fluorophore, chemiluminescent substance, biotin or derivative thereof, avidin or derivative thereof, enzyme, etc. The label may be attached to a carrier constituent or may be carried on a carrier as an independent substance to be delivered.

In the present description, for extracellular matrix-producing cells in the intestine' or for delivery to extracellular matrix-producing cells in the intestine' means that it is suitable for use for extracellular matrix-producing cells in the intestine as target cells, and this includes, for example, it being possible to deliver a substance to these cells, more rapidly, efficiently, and/or in a larger quantity than to other cells, for example, normal cells. For example, the carrier of the present description can deliver a substance to extracellular matrix-producing cells in the intestine at a rate and/or efficiency of 1.1 times or more, 1.2 times or more, 1.3 times or more, 1.5 times or more, 2 times or more, or even 3 times or more compared with other cells.

The present description also relates to a composition for controlling the activity or growth of extracellular matrix-producing cells in the intestine, for treating fibrosis of the intestine, or for regenerating normal tissue of the intestine from fibrotic tissue of the intestine, the composition comprising the above-mentioned carrier and the above-mentioned drug for controlling the activity or growth of extracellular matrix-producing cells in the intestine, and the present description also relates to use of the carrier in the production of said composition. One embodiment of the composition of the present invention contains an effective amount of retinoid for making it target extracellular matrix-producing cells in the intestine. Furthermore, one embodiment of the composition of the present invention is made to target extracellular matrix-producing cells in the intestine by means of a retinoid.

Fibrosis of the intestine in the present description means a pathological condition characterized by excessive deposition of scar tissue on the wall of the intestine and includes chronic inflammation of the intestine such as for example chronic inflammatory bowel disease (a specific inflammatory bowel disease whose cause is identified, such as drug-induced enteritis or infectious enteritis, and a nonspecific inflammatory bowel disease whose cause is unknown, such as Crohn's disease or ulcerative colitis), and one following tissue injury due to radiation, adhesion of the intestine associated with surgery or trauma, etc.

In the present description, regenerating normal tissue of the intestine from fibrotic tissue of the intestine' means recovering tissue of the intestine that has been altered by fibrosis to at least a state with a lesser degree of fibrosis. That is, the tissue of the intestine is replaced by fibrous tissue mainly of the extracellular matrix as fibrosis of the intestine progresses, and reversing this trend and replacing the increased fibrous tissue with the original normal tissue is the regeneration of normal tissue of the intestine from fibrotic tissue of the intestine in the present description. Therefore, regeneration of normal tissue of the intestine from fibrotic tissue of the intestine in the present description includes not only complete recovery of fibrotic tissue of the intestine to the original state but also partial recovery of fibrotic tissue of the intestine to the original state. The degree of regeneration of normal tissue of the intestine may be evaluated based on normalization of tissue structure, reduction of the region occupied by fibrous tissue, increase of the region occupied by normal tissue, etc. by histological examination of a biopsy sample, etc. or may be evaluated by improvement of a biochemical indicator, etc. when an abnormality due to fibril formation in the biochemical indicator, etc. has been observed before treatment with the present composition.

In the composition of the present invention, as long as the retinoid contained in the carrier is present in a mode such that it functions as a targeting agent, the carrier may contain a substance to be delivered within its interior, it may be attached to the exterior of a substance to be delivered, or may be mixed with a substance to be delivered. Therefore, depending on the administration route and the manner in which the drug is released, etc., the composition may be covered with an appropriate material such as, for example, an enteric coating or a timed-disintegration material, or may be incorporated into an appropriate drug release system. Furthermore, the composition of the present invention may be in the form of a complex of a substance to be delivered and a retinoid-coupled liposome, that is, a lipoplex. Moreover, when the carrier is constituted only from a retinoid, the composition of the present invention may be in the form of a complex of the retinoid and a drug for controlling the activity or growth of extracellular matrix-producing cells in the intestine.

The composition of the present invention may be used as a medicine (that is, a pharmaceutical composition) and may be administered via various routes including both oral and parenteral routes; examples thereof include, but are not limited to, oral, intravenous, intramuscular, subcutaneous, local, intrapulmonary, intra-airway, intratracheal, intrabronchial, transnasal, gastric, enteral, intrarectal, intraarterial, intraportal, intraventricular, intramedullary, intra-lymph node, intra-lymphatic, intracerebral, intrathecal, intracerebroventricular, transmucosal, percutaneous, intranasal, intraperitoneal, and intrauterine routes, and it may be formulated into a dosage form suitable for each administration route. Such a dosage form and formulation method may be selected as appropriate from any known dosage form and method (see e.g. Hyojun Yakuzaigaku (Standard Pharmaceutics), Ed. by Yoshiteru Watanabe et al., Nankodo, 2003).

Examples of dosage forms suitable for oral administration include, but are not limited to, powder, granule, tablet, capsule, liquid, suspension, emulsion, gel, and syrup, and examples of dosage forms suitable for parenteral administration include injections such as an injectable solution, an injectable suspension, an injectable emulsion, and an injection to be prepared at the time of use. Formulations for parenteral administration may be in a form such as an aqueous or nonaqueous isotonic sterile solution or suspension.

The present description also relates to a process for producing a pharmaceutical composition for treating fibrosis of the intestine or a composition for regenerating normal tissue of the intestine from fibrotic tissue of the intestine, the process comprising a step of formulating a retinoid as a targeting agent for extracellular matrix-producing cells in the intestine and a drug for controlling the activity or growth of extracellular matrix-producing cells in the intestine as an active ingredient. The method for formulating the retinoid is not particularly limited as long as the retinoid can function as a targeting agent for extracellular matrix-producing cells in the intestine in the composition in which it is formulated, and for example various methods described herein may be used. Furthermore, the method for formulating the active ingredient is not particularly limited as long as the active ingredient can exhibit a predetermined effect, and any known method may be used. Formulation of the active ingredient may be carried out at the same time as formulation of the retinoid or may be carried out before or after formulating the retinoid. For example, when the composition contains a carrier constituent other than the retinoid, formulation of the active ingredient may be carried out such as by mixing the active ingredient with a carrier in which the retinoid has already been formulated as the targeting agent, it may be carried out such as by mixing the retinoid, a carrier constituent other than the retinoid, and the active ingredient at the same time, or it may be carried out such as by formulating the active ingredient with a carrier constituent other than the retinoid and then mixing this with the retinoid.

The formulation amount of retinoid, etc. is as described above with respect to the carrier of the present description. Furthermore, the formulation amount of active ingredient is an amount that, when administered as the composition, can suppress the onset or recurrence of fibrosis of the intestine, improve the clinical condition, alleviate its symptoms, or delay or halt its progression, preferably may be an amount that can prevent the onset or recurrence of fibrosis of the intestine or cure it, or may be an amount that can regenerate normal tissue of the intestine from fibrotic tissue of the intestine. It is also preferably an amount that does not cause an adverse effect that exceeds the benefit from administration. Such an amount may be known or be appropriately determined by an in vitro test using cultured cells or by a test in a model animal such as a mouse, rat, dog, or pig, and such test methods are well known to a person skilled in the art. Examples of model animals with fibrosis of the intestine include those described in Pizarro et al. Trends Mol Med. 2003 May; 9 (5) : 218-22 (e.g. TNBS-induced model, DSS-induced model, oxazolone-induced model, CD4⁺CD45RB^{high}SCID transfer model, tgε26 bone marrow chimera mouse, IL-10 KO mouse, TNF^{ΔARE} model, C3H-HeJBir mouse, SAMP1/Yit mouse, SAMP1/YitFc mouse, etc.). Among them, the SAMP1/Yit mouse is useful as a model mouse for fibrosis of the intestine in human Crohn's disease. The formulation amount of active ingredient can vary according to the form of administration of the composition. For example, when a plurality of units of the composition are used in one administration, the formulation amount of active ingredient in one unit of the composition may be that obtained by dividing the amount of active ingredient required for one administration by the number of units. Such adjustment of the formulation amount may be carried out appropriately by a person skilled in the art.

The carrier or the composition of the present invention may be provided in any form, but from the viewpoint of storage stability, it may preferably be provided in a form that can be prepared at the time of use, for example in a form such that it can be prepared at a place of medical treatment or in the vicinity thereof by a doctor and/or pharmacist, nurse or other paramedic, etc. In this case, the carrier or the composition of the present invention is provided as one or more containers containing at least one component essential therefor, and it is prepared prior to use, for example, within 24 hours prior to use, preferably within 3 hours prior to use, and more preferably, immediately prior to use. When carrying out preparation, a reagent, a solvent, preparation equipment, etc, that are normally available at the place of preparation may be used as appropriate.

Accordingly, the present description also relates to a kit for preparing the carrier or the composition, the kit comprising one or more containers that contain singly or in combination a retinoid, and/or a substance to be delivered, and/or a carrier constituent other than the retinoid, as well as to a component that is necessary for the carrier or the composition provided in the form of such a kit. The kit of the present description may contain, in addition to the above, instructions such as for example a written explanation or an electronic recording medium such as a CD or DVD regarding methods for preparing or administering the carrier and composition of the present description, etc. Furthermore, the kit of the present description may contain all of the components for completing the carrier or the composition of the present description, but need not necessarily contain all of the components, Accordingly, the kit of the present description need not contain a reagent or solvent that is normally available at a place of medical treatment, an experimental facility, etc., such as, for example, sterile water, physiological saline, or glucose solution.

The present description further relates to controlling the activity or growth of extracellular matrix-producing cells in the intestine, for treating fibrosis of the intestine, or for regenerating normal tissue of the intestine from fibrotic tissue of the intestine, comprising administering an effective amount of the above composition to a subject in need thereof. Here, the effective amount in for example a method for treating fibrosis of the intestine is an amount that suppresses the onset or recurrence of fibrosis of the intestine, improves the clinical condition, alleviates its symptoms, or delays or halts its progression, and may preferably be an amount that prevents the onset or recurrence of fibrosis of the intestine or cures it, or may be an amount that can regenerate normal tissue of the intestine from fibrotic tissue of the intestine. It is also preferably an amount that does not cause an adverse effect that exceeds the benefit from administration. Such an amount may be appropriately determined by an in vitro test using cultured cells or by a test in a model animal such as a mouse, rat, dog, or pig, and such test methods are well known to a person skilled in the art. Moreover, the dose of the retinoid contained in the carrier and the dose of the drug used in the method of the present description are known to a person skilled in the art, or may be appropriately determined by the above-mentioned test, etc. A model animal for fibrosis of the intestine is as described above.

The specific dose of the composition administered in the therapeutic use description of the present description may be determined taking into account various conditions with respect to the subject in need of treatment, such as the severity of symptoms, the general health condition of the subject, the age, body weight, and gender of the subject, diet, the administration route, the timing and frequency of administration, concurrent medication, responsiveness to the treatment, compliance with the treatment, etc.

The route of administration includes various routes including both oral and parenteral routes such as, for example, oral, intravenous, intramuscular, subcutaneous, local, intrapulmonary, intra-airway, intratracheal, intrabronchial, transnasal, gastric, enteral, intrarectal, intraarterial, intraportal, intraventricular, intramedullary, intra-lymph node, intra-lymphatic, intracerebral, intrathecal, intracerebroventricular, transmucosal, percutaneous, intranasal, intraperitoneal, and intrauterine routes.

The frequency of administration varies depending on the properties of the composition to be used and the aforementioned conditions of the subject, and may be, for example, a plurality of times per day (more specifically, 2, 3, 4, 5, or more times per day), once a day, every few days (more specifically, every 2, 3, 4, 5, 6, or 7 days, etc.), a few times per week (e.g. 2, 3, 4 times, etc. per week), once a week, or every few weeks (more specifically, every 2, 3, 4 weeks, etc.).

In the present invention, the term 'subject' means any living individual, preferably an animal, more preferably a mammal, and yet more preferably a human individual. In the present description, the subject may be healthy or affected by some disease, and when treatment of fibrosis of the intestine is intended, it typically means a subject affected by fibrosis of the intestine or at risk of being affected thereby. When prevention of fibrosis of the intestine is intended, for example, typical examples include, but are not limited to, a subject affected by a disease that causes fibrosis of the intestine such as an inflammatory bowel disease or adhesion of the intestine associated with surgery or trauma, etc.

Furthermore, the term 'treatment' includes all types of medically acceptable prophylactic and/or therapeutic intervention for the purpose of the cure, temporary remission, or prevention of a disease. For example, the term 'treatment' includes medically acceptable intervention for various purposes, including delaying or halting the progression of fibrosis of the intestine, the regression or disappearance of a lesion, and the prevention of onset and prevention of recurrence of fibrosis of the intestine.

The present description also relates to a method utilizing the above carrier for delivering a substance to extracellular matrix-producing cells in the intestine. This method includes, but is not limited to, for example, a step of loading a substance to be delivered to the carrier, and a step of administering or adding the carrier carrying the substance to be delivered to an organism or a medium, for example a culture medium, which contains extracellular matrix-producing cells of the intestine. These steps may appropriately be achieved according to any known method or a method described herein. The delivery method may be combined with another delivery method, for example, another delivery method for targeting the intestine. Moreover, the method includes an embodiment performed in vitro and an embodiment in which extracellular matrix-producing cells of the intestine inside the body are targeted. The substance that can be transported by the carrier of the present description is as described above.

The present description also relates to an extracellular matrix-producing cell line of the intestine having a vimentin-positive, aSMA-negative, and GFAP-negative phenotype, derived from fibrotic bowel tissue harvested from a subject suffering from fibrosis of the intestine.

Examples of the subject suffering from fibrosis of the intestine include, but are not limited to, a subject diagnosed with fibrosis of the intestine and a model animal with fibrosis of the intestine. Diagnosis of fibrosis of the intestine is carried out based on medical history, confirmation of stricture of the intestine by means of barium imaging, etc., histopathological examination of a biopsy sample, etc. Harvesting of fibrotic bowel tissue may be carried out by any possible method such as surgery or biopsy using an endoscope, etc. A phenotype may be ascertained by, for example, immunostaining by means of an anti-vimentin antibody, an anti-aSMA antibody, and an anti-GFAP antibody, analysis of expression of vimentin, αSMA, and GFAP genes, etc., but is not limited thereto. The antibodies may be commercial products or may be newly prepared by a known method such as immunization of an animal with each protein. The cell line may express HSP47 or a homolog thereof (e.g. gp46), collagen, or a VA storage-related gene such as for example ADRP, LRAT and/or LXRβ. The cell line does not become αSMA- and GFAP-positive when cultured in vitro. The cell line may also be immortalized by transfection of an immortalizing gene (e.g. SV40T, telomerase gene, etc.).

The cell line may be cultured under standard culturing conditions for mesenchymal cells. Examples of such conditions include, but are not limited to, culturing with 10% FBS-containing DMEM and 5% CO₂ at 37°C.

The present description also relates to a method for isolating extracellular matrix-producing cells in the intestine, the method comprising
(i) a step of obtaining cells from fibrotic bowel tissue harvested from a subject suffering from fibrosis of the intestine, and
(ii) a step of selecting cells having a vimentin-positive, αSMA-negative, and GFAP-negative phenotype from the cells obtained in (i).

The acquisition of cells from fibrotic bowel tissue may be carried out by, for example, culturing tissue optionally being finely cut, and obtaining cells migrating therefrom or treating tissue with a protein degradation enzyme (e.g. collagenase, protease, etc.) and obtaining cells separated therefrom, but is not limited thereto.

The selection of cells may be carried out by, for example, separating cells into single cells by a limiting dilution method, etc. and ascertaining the phenotype of each clone by immunostaining, gene expression analysis, etc. or subjecting a suspension of single cells labeled with an anti-vimentin antibody, an anti-aSMA antibody, and/or an anti-GFAP antibody to a cell sorter, etc., but is not limited thereto.

The present description also relates to a method for preparing an extracellular matrix-producing cell line of the intestine, the method comprising
(i) a step of obtaining cells from fibrotic bowel tissue harvested from a subject suffering from fibrosis of the intestine, and
(ii) a step of selecting cells having a vimentin-positive, aSMA-negative, and GFAP-negative phenotype from cells obtained in (i),
the method comprising a step of immortalizing the cells after step (i) or (ii).

Immortalization of cells may be carried out by transfection with an immortalizing gene (e.g. SV40T, a telomerase gene, etc.). Immortalization may be carried out before or after selecting cells having a desired phenotype. The features other than the step of immortalizing cells is as described for the method for isolating extracellular matrix-producing cells in the intestine.

The present description also relates to a method for screening a factor for treating fibrosis of the intestine, the method comprising
(i) a step of making a test factor to coexist with the extracellular matrix-producing cell line of the intestine having a vimentin-positive, aSMA-negative, and GFAP-negative phenotype derived from fibrotic bowel tissue harvested from a subject suffering from fibrosis of the intestine , and
(ii) a step of detecting a change in the cell line due to coexistence with the test factor.

In the present description, the test factor includes a substance such as a compound as well as various factors such as heat, electromagnetic waves (e.g. radio waves, light, X-rays, gamma-rays, etc.), pressure, and pH. Making the cell line 'to coexist' with a test factor means placing the cell line and the test factor in one and the same medium but does not necessarily require contact between the two. Coexistence of the cell line and a test factor includes, but is not limited to, placing the cell line and the test factor in one and the same container. Making a cell line to coexist with a test factor may be carried out in vivo or in vitro.

Examples of the change in the cell line due to coexistence with a test factor include, but are not limited to, inhibition or promotion of an activity of the cell line (e.g. inhibition or promotion of gene expression or substance production) and inhibition or enhancement of proliferation of the cell line. Therefore, for example, inhibition of proliferation of the cell line or inhibition of an activity of the cell line due to coexistence with a test factor represents said test factor being a factor for treating fibrosis of the intestine.

The present description also relates to a kit for screening a factor for treating fibrosis of the intestine, the kit comprising the extracellular matrix-producing cell line of the intestine having a vimentin-positive, aSMA-negative, and GFAP-negative phenotype derived from fibrotic bowel tissue harvested from a subject suffering from fibrosis of the intestine. The present kit may include, in addition to the cell line, a reagent for detecting a change in the cell line, instructions related to a method for screening a factor for treating fibrosis of the intestine using the present kit, such as a written explanation, an electronic recording medium such as a CD or a DVD, etc.

### [Examples]

The present invention is explained in further detail by reference to Examples below, but they are only illustrative.

### Example 1 Identification of stellate cell-like cells in the intestine of SAMP1/Yit Crohn's disease model mouse

In order to examine whether or not cells corresponding to hepatic stellate cells are involved in fibrosis of Crohn's disease, SAMPl/Yit, which is a Crohn's disease model mouse, was used. SAMP1/Yit mice are spontaneous model mice obtained by inbreeding, over 20 generations, mice having an ulcer on the skin among SAMP1 mice, which are prepared by breeding AKR/J mice littermates through 24 generations; they spontaneously produce ileitis at up to 20 weeks old (Matsumoto et. al., Gut. 1998 Jul; 43 (1): 71-8), and are histopathologically characterized by (i) inflammation similar to Crohn's disease commonly occurring at the ileum terminal, (ii) lesions extending discontinuously and transmurally, (iii) observation of thickening of the muscle layer, crypt hyperplasia, villous atrophy, inflammatory cell infiltration into lamina propria and submucosa, hyperplasia of Paneth cells and germ cells, granuloma, and crypt abscess, etc. (Kosiewicz et al., J Clin Invest. 2001 Mar; 107 (6) : 695-702) . Most IBD model mice are models that are affected by colitis, whereas SAMP1/Yit mice having the above characteristics are thought to be model mice having conditions that are the closest to Crohn's disease among existing disease model animals (Pizarro et al., Trends Mol Med. 2003 May; 9 (5) : 218-22) .

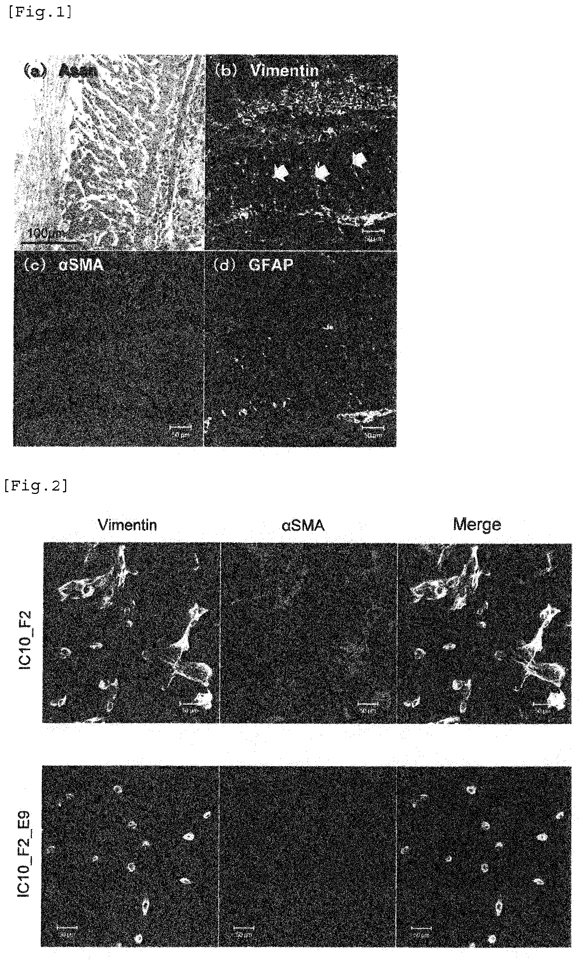

In order to identify stellate cell-like cells in the tissue of the intestine of a SAMP1/Yit mouse, an ileal fibrosis site was examined by an immunohistochemical staining method. First, ileal tissue was harvested from a SAMP1/Yit mouse (29 weeks old, donated by Yakult Central Institute) . The tissue was fixed in 30% neutral formalin for 24 hours, then embedded in paraffin, and sliced thinly to give a sample section. When the sample section was stained with azan and examined for its fibrous state, accumulation of collagen fibers was observed between thickened muscle layer cells (FIG. 1 (a)). Furthermore, sequential sections were subjected to immunohistochemical analysis using antibodies against hepatic stellate cell markers . As the antibodies an anti-vimentin antibody (Anti-vimentin, Abcam, clone RV202, cat. No. ab8978, label: Alexa Fluor 488), an anti-αSMA antibody (Anti-α-Smooth Muscle Actin, SIGMA, clone 1A4, cat. No. A2547, label: Cy3), and an anti-GFAP antibody (Anti-Glial Fibrillary Acidic Protein, Dako, Polyclonal Rabbit, Code No. Z0334, label: DyLight 633) were used. After immunofluorescence staining was carried out by a standard method, when examination with a cofocal laser scanning microscope was carried out, a large amount of localization of cells having a vimentin(+)/αSMA(-)/GFAP(-) phenotype was observed along the accumulated collagen in a fibrotic lesion site of the ileal muscle layer (see arrows in FIG. 1 (b) to (d)). On the other hand, there were no such cells observed in the ileal muscle layer site of an AKR/J mouse, which was a background mouse. These results suggest that quiescent hepatic stellate cell-like cells having a vimentin(+)/αSMA(-)/GFAP(-) phenotype are involved in fibrosis.

### Example 2 Establishment of stellate cell-like cell line from fibrotic small intestine tissue of SAMP1/Yit mouse

In order to subject the cells examined in Example 1 to functional analysis in vitro and, furthermore, examine a therapy for fibrosis of the intestine, etc. utilizing same, stellate cell-like cells having a vimentin(+)/αSMA(-)/GFAP(-) phenotype were separated and cultured from fibrotic small intestine tissue of an SAMP1/Yit mouse to thus establish a stellate cell-like cell line.

First, ileal tissue was harvested from a SAMP1/Yit mouse (21 weeks old), finely cut into about 1 mm length using scissors, then immersed in 20 mL of an EDTA solution (a solution of 4.5 mM EDTA in HBSS (pH 7.5), the same applies below), and lightly shaken. After being allowed to stand at 4°C for 15 minutes, the supernatant was removed, and resuspension was carried out in fresh EDTA solution. After the EDTA solution was exchanged five times, the ileal tissue pieces were suspended in 10% FBS-containing DMEM, plated on a 6 well culture dish, and cultured in 5% CO₂ at 37°C. On the 5th day after starting culturing, a group of cells with a mesenchymal cell-like morphology adhered to the dish and started to proliferate. At this time, transfection of SV40T immortalizing gene was carried out using the retrovirus vector pMFG-tsT-IRES-neo (Kawano et al., Blood. 2003 Jan 15; 101 (2): 532-40), thus giving an IC10_F2 cell line (FIG. 2, top). Subsequently, the IC10_F2 cell line was subjected to a limiting dilution method and immunostaining with anti-αSMA antibody, anti-GFAP antibody, and anti-vimentin antibody to thus attempt to clone cells having a vimentin(+)/αSMA(-)/GFAP(-) phenotype, and as a result an IC10_F2_E9 stellate cell-like cell line derived from the intestine having such a phenotype could be established (FIG. 2, bottom).

Whether or not the established IC10_F2_E9 expressed a group of VA storage-related genes, which are characteristic of stellate cells, was examined using real time PCR. First, total RNA was prepared from each of IC10_F2 cells and IC10_F2_E9 cells using an RNeasy Mini Kit (QIAGEN, 74104), and cDNA was prepared by reacting with a reverse transcriptase (High Capacity RNA-to-cDNA Master Mix, Applied Biosystems, 4390713). The cDNA thus obtained was used to measure the level of expression of a group of vitamin A storage-related genes (ADRP, LRAT and LXRβ) by real time PCR in a LightCycler® 480 system (Roche Applied Science) . As a PCR reagent, LightCycler® 480 Probes Master (Roche Applied Science, 4707494) was used. As probes, those included in the Universal ProbeLibrary Probes (Roche Applied Science) were used (vimentin: Probe #79, 4689020, αSMA: Probe #11, 4685105, ADRP: Probe #79, 4689020, LRAT: Probe #79, 4689020, LXRβ: Probe #106, 4692250).

Furthermore, the primers used were as follows (hereinafter, 'F' denotes a forward primer and 'R' denotes a reverse primer).
Vimentin:
   F 5' TGCGCCAGCAGTATGAAA 3'(SEQ ID NO:1)
   R 5' GCCTCAGAGAGGTCAGCAAA 3'(SEQ ID NO:2)
αSMA:
   F 5' TCACCATTGGAAACGAACG 3'(SEQ ID NO:3)
   R 5' ATAGGTGGTTTCGTGGATGC 3'(SEQ ID NO:4)
ADRP:
   F 5' CCTCAGCTCTCCTGTTAGGC 3'(SEQ ID NO:5)
   R 5' CACTACTGCTGCTGCCATTT 3'(SEQ ID NO:6)
LRAT:
   F 5' GAAGGTGGTCTCCAACAAGC 3'(SEQ ID NO:7)
   R 5' TACTGTGTCCACACGGATGC 3'(SEQ ID NO:8)
LXRβ :
   F 5' GCTCTGCCTACATCGTGGTC 3'(SEQ ID NO:9)
   R 5' CTCATGGCCCAGCATCTT 3'(SEQ ID NO:10)

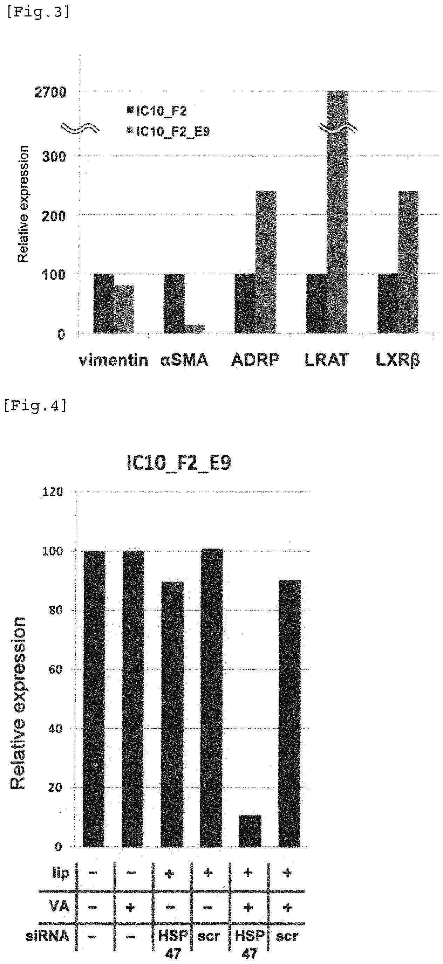

ADRP is one type of PAT (perilipin adipophilin TIP47) protein, which localizes on a lipid droplet membrane and is involved in the biosynthesis or metabolism of lipid droplets (Lee et al., J Cell Physiol. 2010 Jun; 223 (3) : 648-57), LRAT is a retinol esterifying enzyme, localizes on the endoplasmic reticulum membrane in hepatic stellate cells, and is thought to be involved in the storage of VA (Nagatsuma et al., Liver Int. 2009 Jan; 29 (1) : 47-54), and LXRβ is a nuclear receptor involved in lipid metabolism and anti-inflammation and is observed to be expressed at the mRNA level in hepatic stellate cells (Beaven SW. et al., Gastroenterology. 2011 Mar; 140 (3) : 1052-62) . The results show that, compared with the IC10_F2 parent cell line, IC10_F2_E9 exhibited 2.4 times the gene expression for ADRP, 27 times the gene expression for LRAT, and 2.4 times the gene expression for LXRβ (FIG. 3). This result indicates that IC10_F2_E9 has the characteristics of stellate cells.

### Example 3 Preparation of siRNA-containing VA-coupled liposome (1) siRNA

As siRNA targeted to the base sequence of HSP47 (mouse, GenBank Accession No. X60676), which is a common molecular chaperone for collagens (type I to IV), those below were used. A: GGACAGGCCUGUACAACUA (siRNA sense strand sequence starting from the 969th base in the base sequence of mouse HSP47, SEQ ID NO:11) B: UAGUUGUACAGGCCUGUCC (siRNA antisense strand sequence, SEQ ID NO:12)

As siRNArandom (also called siRNAscramble (abbreviation: scr)), which was the control, those below were used.
C: CCUCCAAACCAAUUGGAGG (siRNA sense strand, SEQ ID NO:13)
D: CCUCCAAUUGGUUUGGAGG (siRNA antisense strand, SEQ ID NO:14)

### (2) Preparation of VA-lip-siRNA

As a cationic lipid, a cationic liposome (LipoTrust) containing *O,O*'-ditetradecanoyl-*N*-(α-trimethylammonioacetyl)diethanolamin e chloride (DC-6-14), cholesterol, and dioleylphosphatidylethanolamine (DOPE) at a molar ratio of 4:3:3 was purchased from Hokkaido System Science Co., Ltd. (Sapporo, Japan). Liposome was prepared prior to use at a 1 mM (DC-6-14) concentration by adding, while stirring, doubly distilled water (DDW) to a lyophilized lipid mixture. In order to prepare a VA-coupled liposome, 20 nmol of vitamin A (retinol, Sigma, USA) dissolved in DMSO was mixed with a liposome suspension (20 nmol as DC-6-14) in a 1.5 mL tube at 25°C while stirring. In order to prepare an HSP47 siRNA-carrying VA-coupled liposome (VA-lip-HSP47 siRNA), an HSP47 siRNA solution (3 nmol/mL in DDW) was added to a retinol-coupled liposome solution at room temperature while stirring. The molar ratio of siRNA to DC-6-14 was 1:400. In order to obtain a dose desirable for use in vitro, VA-lip-siRNA was reconstituted using phosphate-buffered physiological saline (PBS) .

### Example 4 Transfection of IC_F2_E9 cells with siRNA and inhibition of HSP47 expression

100 *µ*L of the siRNA-containing VA-coupled liposome obtained in Example 3 was added to a 6 well multi dish (N140675, Nunc™) containing 1 × 10⁴ cells/well of IC_F2_E9 cells in 10% FBS-containing DMEM, incubation was carried out at 37°C in 5% CO₂ for 1 hour, the medium was then changed, and incubation was carried out at 37°C in 5% CO₂ for a further 48 hours. Subsequently, cells were collected, and a total RNA was prepared by the same method as in Example 2. The total RNA thus obtained was reacted with a reverse transcriptase to thus prepare a cDNA, and inhibition of expression of HSP47 was then evaluated using real time PCR. The primers used were as follows.
F: 5' GAAGGCTGTCGCCATCTC 3'(SEQ ID NO:15)
R: 5' CCCAGTCCTGCCAGATGT 3'(SEQ ID NO:16)

It can be seen from the results shown in FIG. 4 that HSP47 gene was expressed in the IC10_F2_E9 cells and expression of said gene was inhibited only by the siRNA-containing VA-coupled liposome. While taking into consideration the fact that siRNA acts within a cell, this result indicates that the IC10_F2_E9 cells have the ability to produce collagen and the retinoid acts as a targeting agent for dramatically promoting the intake of a substance into IC10_F2_E9 cells as extracellular matrix-producing cells of the intestine.

## Claims

1. A pharmaceutical composition for use in treating fibrosis of the intestine, the composition comprising a carrier comprising a retinoid which promotes the delivery of the composition to extracellular matrix-producing cells in the intestine and a drug for controlling the activity or growth of extracellular matrix-producing cells in the intestine, wherein the fibrosis of the intestine is Crohn's disease or ulcerative colitis, and wherein the drug for controlling the activity or growth of extracellular matrix-producing cells in the intestine is HSP47 siRNA.

2. A pharmaceutical composition for use in recovering tissue of the intestine that has been altered by fibrosis to at least a state with a lesser degree of fibrosis, the composition comprising a carrier comprising a retinoid which promotes the delivery of the composition to extracellular matrix-producing cells in the intestine and a drug for controlling the activity or growth of extracellular matrix-producing cells in the intestine, wherein the fibrosis is Crohn's disease or ulcerative colitis, and wherein the drug for controlling the activity or growth of extracellular matrix-producing cells in the intestine is HSP47 siRNA.

3. The pharmaceutical composition for use according to claim 1 or 2, wherein the retinoid comprises retinol.

4. The pharmaceutical composition for use according to any one of claims 1 to 3, wherein the carrier comprises a retinoid and a carrier component other than the retinoid, the molar ratio of the retinoid to the carrier component other than the retinoid being 8:1 to 1:4.

5. The pharmaceutical composition for use according to any one of claims 1 to 4, wherein it is in a form prepared at the time of use.

6. A kit for preparing the pharmaceutical composition according to any one of claims 1 to 5, the kit comprising one or more containers that comprise either singly or in combination the drug for controlling the activity or growth of extracellular matrix-producing cells in the intestine, the retinoid and, as necessary, a carrier constituent other than the retinoid, wherein the drug for controlling the activity or growth of extracellular matrix-producing cells in the intestine is HSP47 siRNA.

7. A process for producing the pharmaceutical composition for use in treating fibrosis of the intestine according to any one of claims 1 and 3 to 5 or a pharmaceutical composition for use in regenerating normal tissue of the intestine from fibrotic tissue of the intestine according to any one of claims 2 to 5, the process comprising a step of formulating a retinoid which promotes the delivery of the composition to extracellular matrix-producing cells in the intestine, and a drug for controlling the activity or growth of extracellular matrix-producing cells in the intestine as an active ingredient, wherein the drug for controlling the activity or growth of extracellular matrix-producing cells in the intestine is HSP47 siRNA.

## Patentansprüche

1. Pharmazeutische Zusammensetzung zur Verwendung in der Behandlung von Darmfibrose, wobei die Zusammensetzung einen Träger umfassend ein Retinoid, welches das Zuführen der Zusammensetzung an extrazelluläre Matrix-produzierende Zellen im Darm fördert, und einen Wirkstoff zum Kontrollieren der Aktivität oder des Wachstums von extrazellulären Matrix-produzierenden Zellen im Darm umfasst, wobei die Darmfibrose Morbus Crohn oder Colitis ulcerosa ist, und wobei der Wirkstoff zum Kontrollieren der Aktivität oder des Wachstums von extrazellulären Matrix-produzierenden Zellen im Darm HSP47 siRNA ist.

2. Pharmazeutische Zusammensetzung zur Verwendung zum Erholen von Darmgewebe, das von Fibrose verändert wurde, zumindest bis zu einem Stadium mit einem geringeren Grad von Fibrose, wobei die Zusammensetzung einen Träger umfassend ein Retinoid, welches das Zuführen der Zusammensetzung an extrazelluläre Matrix-produzierende Zellen im Darm fördert, und einen Wirkstoff zum Kontrollieren der Aktivität oder des Wachstums von extrazellulären Matrix-produzierenden Zellen im Darm umfasst, wobei die Darmfibrose Morbus Crohn oder Colitis ulcerosa ist, und wobei der Wirkstoff zum Kontrollieren der Aktivität oder des Wachstums von extrazellulären Matrix-produzierenden Zellen im Darm HSP47 siRNA ist.

3. Pharmazeutische Zusammensetzung zur Verwendung gemäß Anspruch 1 oder 2, wobei das Retinoid Retinol umfasst.

4. Pharmazeutische Zusammensetzung zur Verwendung gemäß irgendeinem der Ansprüche 1 bis 3, wobei der Träger ein Retinoid und einen Trägerbestandteil ohne dem Retinoid umfasst, wobei das Molverhältnis des Retinoids zum Trägerbestandteil ohne dem Retinoid 8:1 bis 1:4 beträgt.

5. Pharmazeutische Zusammensetzung zur Verwendung gemäß irgendeinem der Ansprüche 1 bis 4, wobei sie in einer Form ist, die zum Zeitpunkt der Verwendung zubereitet wird.

6. Ein Kit zum Zubereiten der pharmazeutischen Zusammensetzung gemäß irgendeinem der Ansprüche 1 bis 5, wobei das Kit einen oder mehrere Behälter umfasst, die entweder einzeln oder in Kombination den Wirkstoff zum Kontrollieren der Aktivität oder des Wachstums von extrazellulären Matrix-produzierenden Zellen im Darm, das Retinoid und, je nach Bedarf, einen Trägerbestandteil neben dem Retinoid umfassen, wobei der Wirkstoff zum Kontrollieren der Aktivität oder des Wachstums von extrazellulären Matrix-produzierenden Zellen im Darm HSP47 siRNA ist.

7. Verfahren zur Herstellung der pharmazeutischen Zusammensetzung zur Verwendung in der Behandlung von Darmfibrose gemäß irgendeinem der Ansprüche 1 und 3 bis 5 oder einer pharmazeutischen Zusammensetzung zur Verwendung zum Regenerieren von normalem Darmgewebe ausgehend von fibrösem Darmgewebe gemäß irgendeinem der Ansprüche 2 bis 5, umfassend einen Schritt des Formulierens eines Retinoids, welches das Zuführen der Zusammensetzung an extrazelluläre Matrix-produzierende Zellen im Darm fördert, und eines Wirkstoffs zum Kontrollieren der Aktivität oder des Wachstums von extrazellulären Matrix-produzierenden Zellen im Darm als ein wirksamer Bestandteil, wobei der Wirkstoff zum Kontrollieren der Aktivität oder des Wachstums von extrazellulären Matrix-produzierenden Zellen im Darm HSP47 siRNA ist.

## Revendications

1. Composition pharmaceutique pour l'utilisation dans le traitement de la fibrose de l'intestin, la composition comprenant un support comprenant un rétinoïde qui favorise la livraison de la composition à des cellules productrices de matrice extracellulaire dans l'intestin et un médicament pour contrôler l'activité ou la croissance de cellules productrices de matrice extracellulaire dans l'intestin, dans laquelle la fibrose de l'intestin est la maladie de Crohn ou la colite ulcérative, et dans laquelle le médicament pour contrôler l'activité ou la croissance de cellules productrices de matrice extracellulaire dans l'intestin est siARN HSP47.

2. Composition pharmaceutique pour l'utilisation dans la récupération de tissu de l'intestin qui a été altéré par la fibrose à au moins un état avec un degré moindre de fibrose, la composition comprenant un support comprenant un rétinoïde qui favorise la livraison de la composition à des cellules productrices de matrice extracellulaire dans l'intestin et un médicament pour contrôler l'activité ou la croissance de cellules productrices de matrice extracellulaire dans l'intestin, dans laquelle la fibrose est la maladie de Crohn ou la colite ulcérative, et dans laquelle le médicament pour contrôler l'activité ou la croissance de cellules productrices de matrice dans l'intestin est siARN HSP47.

3. La composition pharmaceutique pour l'utilisation selon la revendication 1 ou 2, dans laquelle le rétinoïde comprend du rétinol.

4. La composition pharmaceutique pour l'utilisation selon l'une quelconque des revendications 1 à 3, dans laquelle le support comprend un rétinoïde et un composant de support autre que le rétinoïde, le rapport molaire du rétinoïde au composant de support autre que le rétinoïde étant de 8:1 à 1:4.

5. La composition pharmaceutique pour l'utilisation selon l'une quelconque des revendications 1 à 4, laquelle est sous une forme préparée au moment de l'utilisation.

6. Kit pour la préparation de la composition pharmaceutique selon l'une quelconque des revendications 1 à 5, le kit comprenant un ou plusieurs récipients qui comprennent soit seul soit en combinaison le médicament pour contrôler l'activité ou la croissance de cellules productrices de matrice extracellulaire dans l'intestin, le rétinoïde et, si nécessaire, un constituant de support autre que le rétinoïde, dans lequel le médicament pour contrôler l'activité ou la croissance de cellules productrices de matrice extracellulaire dans l'intestin est siARN HSP47.

7. Procédé pour la production de la composition pharmaceutique pour l'utilisation dans le traitement de la fibrose de l'intestin selon l'une quelconque des revendications 1 et 3 à 5 ou d'une composition pharmaceutique pour l'utilisation dans la régénération de tissu normal de l'intestin à partir de tissu fibreux de l'intestin selon l'une quelconque des revendications 2 à 5, le procédé comprenant une étape de formulation d'un rétinoïde qui favorise la livraison de la composition à des cellules productrices de matrice extracellulaire dans l'intestin, et d'un médicament pour contrôler l'activité ou la croissance de cellules productrices de matrice extracellulaire dans l'intestin en tant qu'ingrédient actif, dans lequel le médicament pour contrôler l'activité ou la croissance de cellules productrices de matrice extracellulaire dans l'intestin est siARN HSP47.
